# EUROPÄISCHE PATENTSCHRIFT

(11) **EP 1 309 816 B1**
(45) Veröffentlichungstag und Bekanntmachung des Hinweises auf die Patenterteilung: **23.11.2005**
(21) Anmeldenummer: 01969346.4
(22) Anmeldetag: 06.07.2001
(51) Int. Cl.: F16M 11/04

(54) **TRÄGER ZUM TRAGEN VON MEDIZINISCHEN APPARATEN**
SUPPORT FOR MEDICAL EQUIPMENT
SUPPORT POUR APPAREILS MEDICAUX

(30) Priorität: 17.08.2000 DE 10040338; 06.09.2000 DE 10043895
(43) Veröffentlichungstag der Anmeldung: 14.05.2003
(73) Patentinhaber: Mavig GmbH, 81829 München (DE)
(72) Erfinder: KUHN, Peter, 81545 München (DE)
(74) Vertreter: Körfer, Thomas
(86) Internationale Anmeldenummer: PCT/EP2001/007783
(87) Internationale Veröffentlichungsnummer: WO 2002/014734

(56) Entgegenhaltungen:
- EP-A- 0 516 475
- WO-A-98/52484
- CH-A- 648 466
- DE-U- 20 015 398
- DE-U- 29 706 015

## Beschreibung

Die Erfindung betrifft einen Träger zum Tragen von medizinischen Apparaten, insbesondere von Bildschirm-Sichtgeräten im Rahmen medizinischer Anwendungen.

Zum Tragen von medizinischen Apparaten werden in Krankenhäusern, Arztpraxen und ähnlichen Einrichtungen Träger verwendet, die über einen Federarm an der Decke des jeweiligen Behandlungsraums bzw. Operationssaals aufgehängt sind. Dadurch ist gewährleistet, daß der Boden des Behandlungsraumes bzw. Operationssaals stets frei zugänglich und insbesondere einfach zu reinigen und zu desinfizieren ist, so daß die gestellten Hygieneanforderungen erfüllt werden können. Der bekannte Träger besteht aus einem C-förmigen Tragarm und einem an diesem befestigten Querträger. Der bekannte Träger ist für einen bestimmten Apparateaufbau konzipiert. Im folgenden ist der Träger, der für einen Aufbau bestehend aus drei Bildschirm-Sichtgeräten konzipiert ist, näher beschrieben. Die Bildschirm-Sichtgeräte haben eine definierte Größe und Gewichtsverteilung. Davon ausgehend werden die geometrischen Verhältnisse und Gewichtsverteilungen des Trägers festgelegt, so daß der an einem festen Aufhängepunkt an dem Federarm aufgehängte Träger die an dem Träger befestigten Bildschirm-Sichtgeräte trägt, ohne daß sich der Tragarm wesentlich neigt oder wesentliche Drehmomentkräfte auf den Aufhängepunkt einwirken.

Der bekannte Träger hat mehrere Nachteile. Da der Träger auf der Grundlage der Gewichtsverteilung und der Größe der vorgegebenen Bildschirm-Sichtgeräte konstruiert ist, ist eine Verwendung des Trägers für andere Bildschirm-Sichtgeräte oder gar andere medizinische Apparate ausgeschlossen. Außerdem stellt sich bei einer wesentlichen Änderung des Aufbaus der zugrunde liegenden Bildschirm-Sichtgeräte, z.B. infolge einer Produktverbesserung oder Produktionsänderung, das Problem, daß eine konstruktive Anpassung des Trägers erforderlich ist, was aber aufwendig ist, die Produktionszahlen eines vorgegebenen Trägertyps verringert und erhebliche praktische Nachteile mit sich bringt. Außerdem ist der bekannte Träger völlig ungeeignet, wenn die zu tragenden medizinischen Apparate nicht von vornherein bestimmt sind und insbesondere wenn mehrere verschiedene medizinische Apparate wechselweise je nach Anwendungseinsatz auf dem Träger befestigt werden.

Aus der DE-U-29 706 015 ist ein Träger mit einem Tragarm und eine Befestigungseinrichtung bekannt, wobei die Eingriffsstelle der Befestigungsvorrichtung an einem Verbindungsabschnitt des Tragarms eingreift.

Der Erfindung liegt daher die Aufgabe zugrunde, einen Träger zu schaffen, der für verschiedene Apparate und für eine unterschiedliche Anzahl von Apparaten geeignet ist, bei dem ein Apparat ausgetauscht werden kann und bei dem insbesondere die Neigung bzw. das auf den Träger einwirkende Drehmoment einstellbar ist.

Die Aufgabe wird durch einen Gegenstand mit den Merkmalen des Anspruches 1 gelöst.

Der erfindungsgemäße Träger hat den Vorteil, daß die Eingriffsstelle, an der die Befestigungseinrichtung zum Aufhängen des Trägers in einen Verbindungsabschnitt des Tragarms eingreift, veränderbar ist. Dadurch kann der Schwerpunkt des beladenen Tragarms bei einer vorgegebenen Neigung, die z.B. durch eine waagrechte Ausrichtung des Apparats gegeben ist, zumindest näherungsweise unter den Aufhängepunkt verschoben werden, indem die Eingriffsstelle verändert wird. Dadurch kann ein auf den Träger einwirkendes Drehmoment verhindert und gegebenenfalls die Neigung des Tragarms eingestellt werden.

Vorteilhafte Weiterbildungen des durch die Merkmale des Anspruches 1 gegebenen Gegenstand sind durch die in den Unteransprüchen angeführten Maßnahmen möglich.

Vorteilhaft ist es, daß der Tragarm bogenförmig, insbesondere C-bogenförmig, ausgebildet ist. Dadurch kann der Tragarm um die Apparate herumgeführt werden, so daß der für die Apparate vorgesehene Raum optimal genutzt werden kann.

Außerdem ist dadurch der Verbindungsabschnitt des Tragarms auf einfache Weise, nämlich durch den oberen zumindest teilweise waagrecht verlaufenden Bogen des C-Bogens des Tragarms gegeben.

Vorteilhaft ist es, daß die Befestigungseinrichtung ein Gelenk, insbesondere ein Kugelgelenk, zur zumindest bezüglich einer Achse drehmomentfreien Aufhängung des Trägers aufweist. Für den Fall, daß der Träger bezüglich der mittels der Wahl der Eingriffsstelle vorgegebenen Aufhängung ungleichmäßig belastet ist, stellt sich infolge der Beweglichkeit aufgrund des Gelenks eine Neigung des Tragarms ein, die von der vorgegebenen Neigung abweicht. Durch Verändern der Eingriffsstelle, an der die Befestigungseinrichtung in den Verbindungsabschnitt des Tragarms eingreift, läßt sich die Neigung des Tragarms auf die vorgegebene Neigung justieren. Somit läßt sich die gewünschte Neigung einstellen, wobei stets eine drehmomentfreie Aufhängung des Trägers gegeben ist.

In vorteilhafter Weise weist zumindest der Verbindungsabschnitt des Tragarms ein Hohlprofil auf, wodurch zumindest im Bereich des Verbindungsabschnitts ein Innenraum ausgebildet ist. Der Verbindungsabschnitt weist dabei zumindest zwei gegenüberliegende Aussparungen auf, durch die die Befestigungseinrichtung in den Innenraum des Verbindungsabschnitts des Tragarms eingreift. Dadurch kann ein wesentlicher Teil der zum Verändern der Eingriffsstelle, an der die Befestigungseinrichtung in den Verbindungsabschnitt des Tragarms eingreift, vorgesehenen Befestigungseinrichtung in dem Innenraum des Verbindungsabschnitts des Tragarms angeordnet werden, so daß sich ein kompakter Aufbau ergibt.

In vorteilhafter Weise ist in.dem Innenraum des Verbindungsabschnitts des Tragarms zum Feststellen der Befestigungseinrichtung bezüglich des Tragarms eine Feststellvorrichtung der Befestigungseinrichtung angeordnet. Dadurch kann nach einer erfolgreichen Verstellung der Befestigungseinrichtung bezüglich des Tragarms des Trägers ein Feststellen der Befestigungseinrichtung bezüglich des Tragarms erfolgen, wobei die Feststellvorrichtung unauffällig und platzsparend in dem Tragarm des Trägers untergebracht ist.

Vorteilhaft ist es, daß die Feststellvorrichtung zumindest eine Feststellplatte aufweist, die zum Feststellen von der Seite des Innenraums des Verbindungsabschnittes gegen eine Wand des Verbindungsabschnittes mit einer Feststellkraft beaufschlagbar ist. Dabei ist es besonders vorteilhaft, daß der Feststellplatte eine bezüglich der Wand des Verbindungsabschnittes zumindest im wesentlichen gegenüberliegende weitere Feststellplatte zugeordnet ist, wobei zum Feststellen der Befestigungseinrichtung bezüglich des Tragarms die beiden Feststellplatten gegeneinander mit der Feststellkraft beaufschlagbar sind. Dadurch wirkt die Feststellkraft über eine große Fläche auf die beteiligten Bauteile ein, so daß aufgrund des kleinen Anpreßdruckes, d.h. aufgrund der großflächigen Kraftverteilung, eine gute Lösbarkeit des Feststellens auch nach einem Feststellen über einen größeren Zeitraum gut möglich ist. Außerdem können die Feststellplatten als integraler Bestandteil der Feststellvorrichtung, d.h. als Verbindungselemente zwischen Bauteilen der Feststellvorrichtung, zum Einsatz kommen.

In vorteilhafter Weise weist die Feststellvorrichtung eine Feststellschraube auf, die in ein Gewinde der Feststellplatte eingreift und die sich beim Festziehen zumindest mittelbar an der weiteren Feststellplatte abstützt. Dadurch wird die Wand des Verbindungsabschnittes des Tragarms, die beidseitig von den Feststellplatten beaufschlagt wird, jeweils mit der gleichen Kraft beaufschlagt, so daß eine Verformung der Wand des Verbindungsabschnittes, insbesondere im Bereich der sich gegenüberliegenden Aussparungen, verhindert ist.

Vorteilhaft ist es, daß die Eingriffsstelle, an der die Befestigungseinrichtung in den Verbindungsabschnitt des Tragarms eingreift, kontinuierlich veränderbar ist. Hierfür ist es insbesondere von Vorteil, daß die Veränderung der Eingriffsstelle mittels einer Spindel erfolgt, die in dem Tragarm gelagert ist und längs der Befestigungseinrichtung verschiebbar ist. Dadurch kann eine stufenlose und genaue Einstellung der Eingriffsstelle erreicht werden, um den Schwerpunkt des beladenen Tragarms bei einer vorgegebenen Neigung des Tragarms zumindest näherungsweise unter den Aufhängepunkt zu verschieben. Durch die Spindel ist dabei eine vorteilhafte Kraftübertragung gewährleistet, die zudem ein besonders einfaches Positionieren der Eingriffsstelle ermöglicht.

Ferner ist es vorteilhaft, daß Zusatzgewichte an dem Tragarm anbringbar sind, um den Schwerpunkt des beladenen Tragarms bei einer vorgegebenen Neigung des Tragarms fein justiert unter den Aufhängepunkt zu verschieben. Dadurch können in einfacher Weise kleine Verstellungen bewerkstelligt werden. Ist es z.B. erforderlich, daß einer der Apparate häufiger ausgetauscht wird, wodurch sich eine andere Gewichtsverteilung ergibt, dann kann dies mit den Zusatzgewichten kompensiert werden, ohne daß eine Veränderung der Eingriffsstelle, an der die Befestigungseinrichtung in den Verbindungsabschnitt des Tragarms eingreift, erforderlich ist.

Ein bevorzugtes Ausführungsbeispiel der Erfindung ist in der Zeichnung vereinfacht dargestellt und in der nachfolgenden Beschreibung näher erläutert. Es zeigen:
- Fig. 1: eine Seitenansicht des Trägers gemäß einem bevorzugten Ausführungsbeispiel;
- Fig. 2: eine Vorderansicht des Trägers gemäß dem bevorzugten Ausführungsbeispiel;
- Fig. 3: den in Fig. 1 mit III bezeichneten Ausschnitt des Trägers in einer auszugsweisen Schnittdarstellung; und
- Fig. 4: den Träger gemäß dem bevorzugten Ausführungsbeispiel in einer Schnittdarstellung entlang der in Fig. 3 mit IV bezeichneten, entlang der Punkte A bis K geführten Schnittlinie.

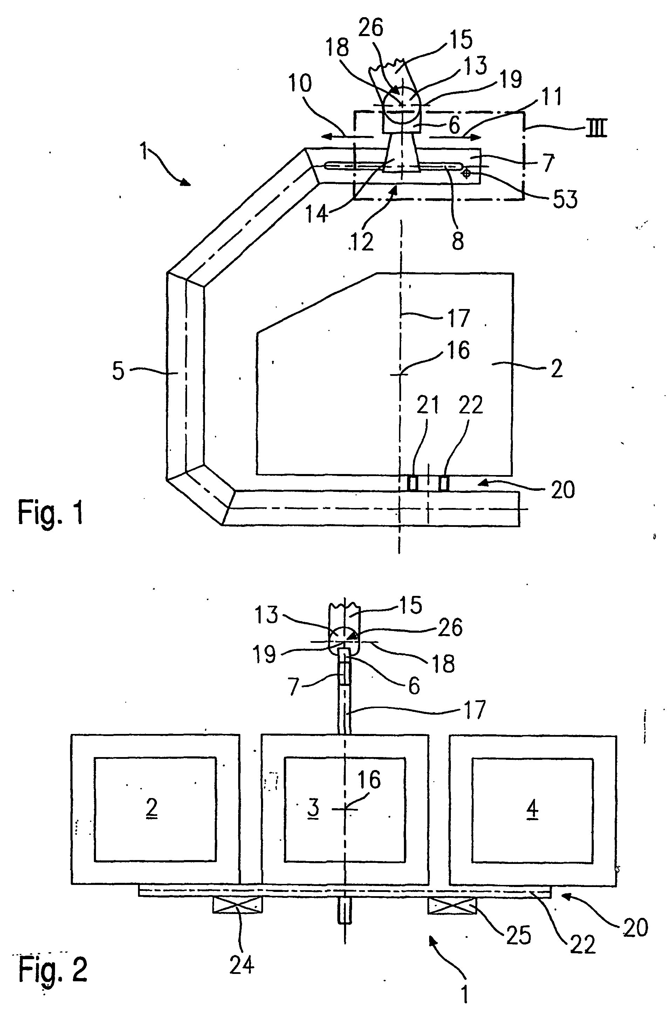

Fig. 1 zeigt ein bevorzugtes Ausführungsbeispiel des erfindungsgemäßen Trägers 1 zum Tragen von zumindest einem Apparat 2. Der Träger 1 dient insbesondere zum Tragen von Bildschirm-Sichtgeräten und anderen medizinischen Geräten. Der bevorzugte Einsatzbereich des Trägers 1 liegt im medizinischen Bereich. Das heißt, der Träger 1 kommt vorzugsweise in Klinikräumen, Arztpraxen und dergleichen zum Einsatz. Der erfindungsgemäße Träger 1 eignet sich jedoch auch für andere Anwendungsfälle.

Der Träger 1 weist einen Tragarm 5 und eine Befestigungseinrichtung 6 auf. Der Tragarm 5 ist C-bogenförmig ausgebildet, wobei im oberen Bereich des Tragarms 5 ein Verbindungsabschnitt 7 vorgesehen ist, in den die Befestigungseinrichtung 6 eingreift. Der Eingriff erfolgt dabei durch zwei sich gegenüberliegende Aussparungen 8,9, von denen in der Fig. 1 die Aussparung 8 dargestellt ist. Die Befestigungseinrichtung 6 ist entlang der Aussparungen 8, 9 in den Richtungen 10, 11 bezüglich des Verbindungsabschnittes 7 des Tragarms 5 verschiebbar, wodurch die Eingriffsstelle 12, an der die Befestigungseinrichtung 6 durch die Aussparungen 8, 9 in den Verbindungsabschnitt 7 des Tragarms 5 eingreift bezüglich der Aussparungen 8, 9 veränderbar ist.

Die Befestigungseinrichtung 6 weist ein Kugelgelenk 13 auf, bezüglich dem ein erster Abschnitt 14 der Befestigungseinrichtung 6 bezüglich des zweiten Abschnitts 15 der Befestigungseinrichtung 6 drehbar ist. Der zweite Abschnitt 15 kann z.B. an einem an der Decke eines Raumes befestigten Federarm befestigt werden. Eine Neigung des Federarms bezüglich der Horizontalen wird dabei durch das Kugelgelenk 13 ausgeglichen. Anstelle des Kugelgelenks 13 kann auch ein einfaches Gelenk, das nur bezüglich einer Achse drehbar ist, verwendet werden. Es können auch andere Gelenke und eine Kombination von mehreren Gelenken anstelle des Kugelgelenks 13 verwendet werden.

Durch das Kugelgelenk 13 erfolgt die Aufhängung des mit den Apparaten 2, 3, 4 belasteten Tragarms 5 drehmomentfrei, so daß der Schwerpunkt 16, der an der Befestigungseinrichtung 6 angehängten Bauteile; die die Bauteile 2 bis 5 umfassen, auf der durch den Mittelpunkt des Kugelgelenks 13 definierten Vertikalachse zum Liegen kommt. Als Drehachsen lassen sich z.B. die Achsen 18, 19 angeben, deren Schnittpunkt im Aufhängepunkt 26 liegt.

Der Tragarm 5 ist mit einem Querträger 20 verbunden, der die beiden Streben 21, 22 umfaßt, die jeweils ein rechteckiges Profil haben. Auf dem Querträger 20 des Trägers 1 sind die Apparate 2, 3, 4 mittels einer üblichen Befestigung befestigt. Die Befestigung kann z.B. durch Festschrauben der Apparate 2, 3, 4 an dem Tragarm 5 erfolgen.

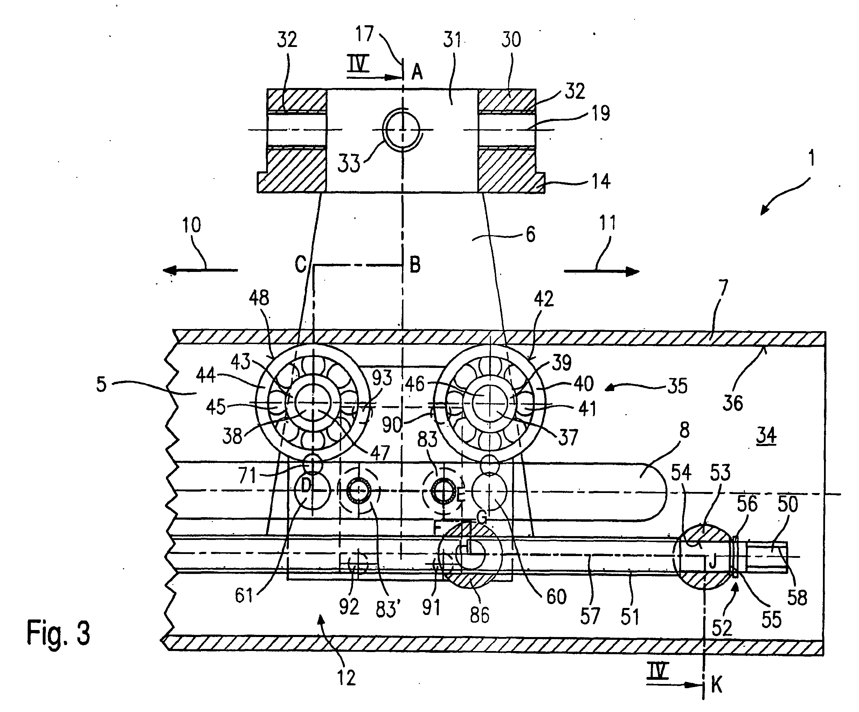

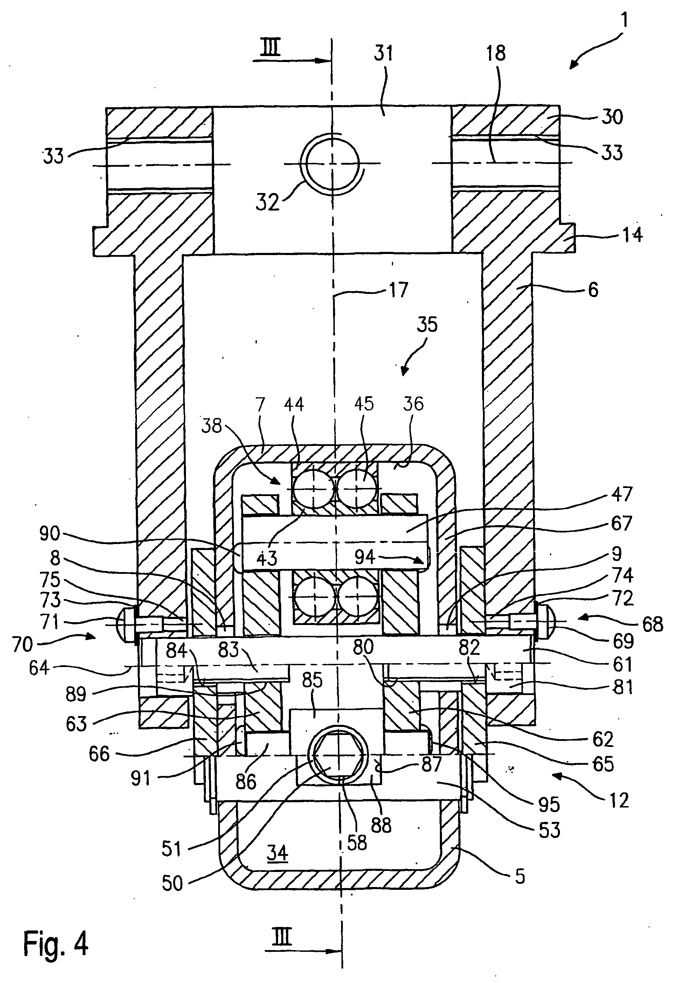

In Fig. 1 ist außerdem ein an dem Verbindungsabschnitt 7 des Tragarms 5 angeordneter Lagerbolzen 53 eines Spindellagers für einen Spindelmechanismus dargestellt, der nachfolgend unter Bezugnahme auf die Fig. 3 und 4 näher beschrieben ist.

Fig. 2 zeigt den Träger 1 gemäß dem bevorzugten Ausführungsbeispiel zum Tragen der Apparate 2, 3, 4 in einer Vorderansicht. Bereits beschriebene Elemente sind in allen Figuren mit übereinstimmenden Bezugszeichen versehen, wodurch sich eine wiederholende Beschreibung erübrigt.

In Fig. 2 sind an den Träger 1 die Zusatzgewichte 24, 25 befestigt, mittels der bei der vorgegebenen Neigung des Tragarms der Schwerpunkt 16 fein justiert unter den im Kreuzpunkt der Achsen 18, 19 liegenden Aufhängepunkt 26 verschoben werden kann. Insbesondere läßt sich durch die Zusatzgewichte 24, 25 eine Veränderung der Neigung des mit den Apparaten 2, 3, 4 beladenen Tragarms 5 bezüglich der Achse 19 erreichen. Dies kann z.B. dann erforderlich sein, wenn die Apparate 2, 4 in unterschiedlichem Abstand zu der Mitte des Querträgers 20, wo der Querträger 20 mit dem Tragarm 5 verbunden ist, angeordnet sind und/oder die Apparate 2, 4 ein unterschiedliches Gewicht haben oder auch falls der Apparat 3 bezüglich seines Schwerpunktes nicht mittig auf dem Querträger 20 befestigt ist.

Die Zusatzgewichte 24, 25 zeichnen sich durch leichte Anbringbarkeit und Entfernbarkeit aus, so daß insbesondere kurzfristig auftretende Schwerpunktveränderungen, die z.B. dadurch auftreten, daß auf den Apparat 2 ein weiterer kleiner Apparat gestellt wird, kurzfristig ausgeglichen werden können.

Fig. 3 zeigt den in Fig. 1 mit III bezeichneten Ausschnitt des Trägers 1 gemäß dem bevorzugten Ausführungsbeispiel in einer Schnittdarstellung.

Die Befestigungseinrichtung 6 des Trägers 1 weist ein Verbindungsteil 30 zum Befestigen des Kugelgelenks 13 oder dergleichen auf. In dem bevorzugten Ausführungsbeispiel weist das Verbindungsteil 30 die jeweils von der Aussparung 31 unterbrochenen Gewinde 32, 33 auf.

Die Befestigungseinrichtung 6, die durch die Aussparungen 8, 9 in den Innenraum 34 des Verbindungsabschnitts 7 des Tragarms 5 eingreift, weist eine in dem Innenraum 34 angeordnete Feststellvorrichtung 35 zum Feststellen der Befestigungseinrichtung 6 bezüglich des Tragarms 5 auf. Hierfür ist der Tragarm 5, insbesondere der Verbindungsabschnitt 7 des Tragarms 5, als Hohlkörper ausgebildet, wobei dieser ein Hohlprofil aufweist, wodurch der Innenraum 34 insbesondere im Bereich des Verbindungsabschnittes 7 ausgebildet ist. Die Feststellvorrichtung 35 ist in dem bevorzugten Ausführungsbeispiel in dem Bereich angeordnet, in dem sich die Eingriffsstelle 12 befindet, an der die Befestigungseinrichtung 6 in den Verbindungsabschnitt 7 des Tragarms 5 eingreift. Der Tragarm 5 weist an dem Verbindungsabschnitt 7 auf der Seite des Innenraums 34 eine Lauffläche 36 auf, an der Laufräder 37, 38 bei einer Verschiebung der Feststellvorrichtung 35 der Befestigungseinrichtung 6 bezüglich des Tragarms 5 abrollen. Über die Laufräder 37, 38 wird die Gewichtskraft des mit den Apparaten 2, 3, 4 beladenen Tragarms 5 auf die Befestigungseinrichtung 6 übertragen.

Das Laufrad 37 weist eine innere Lagerschale 39 und eine äußere Lagerschale 40 auf, zwischen denen Lagerkugeln 41 angeordnet sind, wodurch ein Kugellager gebildet ist. Die innere Lagerschale 39 des Laufrads 37 ist mit einem Bolzen 46 verbunden und an der äußeren Lagerschale 40 ist eine Laufradfläche 42 ausgebildet, an der das Laufrad 37 an der Lauffläche 36 des Verbindungsabschnitts 7 abrollt.

Entsprechend dem Laufrad 37 ist auch das Laufrad 38 aufgebaut. Das Laufrad 38 weist eine innere Lagerschale 43, eine äußere Lagerschale 44 und zwischen diesen angeordnete Lagerkugeln 45 auf, wodurch ein Kugellager gebildet ist. Die innere Lagerschale 43 ist mit dem Bolzen 47 verbunden und an der äußeren Lagerschale 44 ist eine Laufradfläche 48 ausgebildet, die an der Lauffläche 36 des Verbindungsabschnitts 7 beim Verschieben der Befestigungseinrichtung 6 bezüglich des Verbindungsabschnitts 7 des Tragarms 5 abrollt.

Die Verschiebung der Feststellvorrichtung 35 bezüglich des Tragarms 5 erfolgt über eine Spindel 50, die ein Spindelgewinde 51 und einen Lagerabschnitt 52 aufweist. An dem Lagerabschnitt 52 ist die Spindel 50 mittels eines Lagerbolzens 53 in dem Verbindungsabschnitt 7 des Tragarms 5 gelagert. Hierfür weist der Lagerbolzen 53 eine Querbohrung 54 auf, durch die der gewindelose Lagerabschnitt 52 der Spindel 50 geführt ist.' Mittels eines in einem radial umlaufenden Einstich 55 eingebrachten Sperrings 56 ist die Spindel 50 in einer Richtung, die parallel zu der Spindelachse 57 ist, gesichert. Die Spindel 50 weist außerdem an ihrem Lagerabschnitt 52 einen Außensechskant 58 auf, an dem ein geeignetes Werkzeug bzw. eine geeignete Stellvorrichtung zum Betätigen der Spindel 50 anbringbar ist. Beim Betätigen der Spindel 50 wird diese um ihre Spindelachse 57 gedreht, Wodurch das Spindelgewinde 51 je nach Betätigungsrichtung gedreht wird. Das Werkzeug bzw. die Betätigungseinrichtung kann auch anders als über den Außensechskant 58 an der Spindel 50 angreifen. Zum Beispiel kann auch ein Innensechskant an der Spindel 50 im Bereich des Lagerabschnitts 52 oder eine andere Kraft bzw. formschlüssige Verbindung vorgesehen sein.

Im folgenden wird der Träger 1 des bevorzugten Ausführungsbeispiels unter zusätzlicher Bezugnahme auf die Fig. 4 näher beschrieben. Dabei zeigt Fig. 4 einen Schnitt entlang der in Fig. 3 mit IV bezeichneten Schnittlinie, die entlang der Punkte A bis K geführt ist. Ferner ergibt sich die Fig. 3 auch als Schnitt entlang der in Fig. 4 mit III bezeichneten Schnittlinie.

Die Befestigungseinrichtung 6 weist Bolzen 60, 61 auf, mit denen der erste Abschnitt 14 der Befestigungseinrichtung 6, der sich außerhalb des Innenraums 34 befindet, mit der Feststellvorrichtung 35 verbunden ist. Ferner sind Feststellplatten 62, 63 vorgesehen, die auf den Bolzen 60, 61 in einer Richtung parallel zu der Bolzenachse 64 des Bolzens 61 geführt sind. Die Feststellplatten 62, 63 sind daher in einer Richtung, die parallel zu der Bolzenachse 64 des Bolzens 61 ist, bewegbar. In den Feststellplatten 62, 63 ist der Bolzen 47 gelagert, auf dem das Laufrad 38 angeordnet ist. Außerdem ist in den Feststellplatten 62, 63 der Bolzen 46 gelagert, auf dem das Laufrad 37 angeordnet ist. Dabei sind die Bolzen 46, 47 in einer Richtung, die parallel zu der Bolzenachse 64 des Bolzens 61 ist, in den Feststellplatten 62, 63 bewegbar geführt. Die Feststellplatten 62, 63 sind im Innenraum 34 des Verbindungsabschnittes 7 angeordnet. Die Befestigungseinrichtung 6 weist ferner die Feststellplatten 65, 66 auf, wobei die Feststellplatte 65 bezüglich der Wand 67 der Feststellplatte 62 gegenüberliegt und die Feststellplatte 66 bezüglich der Wand 67 der Feststellplatte 63 gegenüberliegt. Die Feststellplatten 65, 66 sind dabei auf den Bolzen 60, 61 in einer Richtung parallel zu der Bolzenachse 64 des Bolzens 61 beweglich gelagert. Um ein Lösen bzw. Verschieben der Bolzen 60, 61 bezüglich des ersten Abschnitts 14 der Befestigungseinrichtung 6, der sich außerhalb des Innenraums 34 des Verbindungsabschnittes 7 befindet, zu verhindern, sind Sicherungsschrauben 68 bis 71 vorgesehen, die zur Selbstsicherung mit Federelementen 72, 73 in Gewindebohrungen 74, 75 des ersten Abschnitts 14 der Befestigungseinrichtung 6 eingeschraubt sind.

Die Feststellvorrichtung 35 weist eine in ein Gewinde 80 der Feststellplatte 62 eingeschraubte Feststellschraube 81 auf, die teilweise in der Aussparung 9 des Verbindungsabschnitts 7 angeordnet ist und die durch eine vorzugsweise als Bohrung ausgebildete Aussparung 82 geführt ist..Beim Anziehen der Feststellschraube 81 stützt sich diese an der Feststellplatte 65 ab, so daß die beiden Feststellplatten 62, 65 aufeinander zu bewegt werden. Zum Feststellen wird mittels der Feststellschraube 81 eine Feststellkraft auf die Feststellplatten 62, 65 übertragen, so daß diese von beiden Seiten gegen die Wand 67 des Verbindungsabschnitts 7 gepreßt werden. Durch die dabei auftretenden Haftreibungskräfte wird eine lösbare Verbindung zwischen der Befestigungseinrichtung 6 und dem Verbindungsabschnitt 7 des Tragarms 5 erreicht.

In entsprechender Weise greift eine Feststellschraube 83 in ein Gewinde 89 der' Feststellplatte 63 ein, wobei sich die Feststellschraube 83 beim Anziehen an der Feststellplatte 66 abstützt und durch eine Aussparung 84 in der Feststellplatte 66 geführt ist. Dabei ist die Feststellschraube 83 teilweise in der Aussparung 8 des Verbindungsabschnittes 7 des Tragarms 5 angeordnet. Indem die Feststellschraube 83 mit einer Feststellkraft angezogen wird, werden die Feststellplatten 63, 66 mit einer Feststellkraft beaufschlagt, wodurch eine auf den auftretenden Haftreibungskräften zwischen den Feststellplatten 63, 66 und der Wand 67 des Verbindungsabschnitts 7 beruhende lösbare Verbindung zwischen der Feststellvorrichtung 35 der Befestigungseinrichtung 6 und dem Verbindungsabschnitt 7 des Tragarms 5 geschaffen wird.

Wenn die beiden Feststellschrauben 81, 83 mit einer Feststellkraft beaufschlagt sind, dann ist die Feststellvorrichtung 35 bezüglich des Verbindungsabschnitts 7 des Tragarms 5 festgestellt. Durch Lösen der Feststellschrauben 81, 84 wird die festgestellte Feststellvorrichtung 35 gelöst, so daß die Feststellvorrichtung 35 der Befestigungseinrichtung 6 bezüglich des. Verbindungsabschnitts 7 des Tragarms 5 verschiebbar ist.

Zum Verschieben der Feststellvorrichtung 35 dient die Spindel 50, die durch eine Spindelführung 85 der Feststellvorrichtung 35 geführt ist. Die Spindelführung 85 ist an einem quer zur Spindelachse 57 orientierten Haltebolzen 86 befestigt, wobei auf dem Haltebolzen 86 die Feststellplatten 62, 63 in einer Richtung, die parallel zu der Bolzenachse 64 des Bolzens 61 ist, bewegbar geführt sind. Bei einer Betätigung der Spindel 50 rotiert diese um die Spindelachse 57, so daß je nach Rotationsrichtung die Feststellvorrichtung 35 und damit die gesamte Befestigungseinrichtung 6 relativ zu dem Verbindungsabschnitt 7 in der Richtung 10 oder 11 verschoben wird. Dadurch kann die Eingriffsstelle 12, an der die Befestigungseinrichtung 6 in den Verbindungsabschnitt 7 des Tragarms 5 eingreift, mittels der Spindel 50 kontinuierlich verändert werden.

In der quer zur Längsachse des Lagerbolzens 53 ausgebildeten Aussparung 87 ist ein Lagerelement 88 vorgesehen, in dem die Querbohrung 54 ausgebildet ist. Die Spindel 50 wird an ihrem einen Ende in der Querbohrung 54 des Lagerbolzens 53 und in der Spindelführung 85 geführt. Gegebenenfalls kann auch eine weitere Führung, die der Führung mittels des Lagerelements 88 bzw. der Spindelführung 85 entspricht, an dem anderen Ende der Spindel 50 vorgesehen sein.

In der obigen Beschreibung ist die Feststellung der Feststellvorrichtung 35 mittels der Feststellschrauben 81, 83 ausführlich beschrieben. Um eine gleichmäßige Beaufschlagung der Feststellplatten 62, 63, 65, 66 zu erreichen, sind neben den bereits beschriebenen Feststellschrauben 81, 83 zwei weitere Feststellschrauben vorgesehen. In der Fig. 3 ist neben der Feststellschraube 83 auch die Feststellschraube 83' angeordnet. Die noch verbleibende Feststellschraube ist symmetrisch bezüglich der in Fig. 4 dargestellten mit III bezeichneten Schnittlinie zu der Feststellschraube 83' angeordnet.

In dem bevorzugten Ausführungsbeispiel sind an der Feststellplatte 63 Reibungskörper 90 bis 93 vorgesehen, die beim Feststellen der Feststellvorrichtung 35 durch die Feststellkräft gegen die Wand 67 des Verbindungsabschnitts 7 des Tragarms 5 gedrückt werden, um die erforderliche Haftreibungskraft aufzubringen. Entsprechend sind auch an der Feststellplatte 62 vier Reibungskörper vorgesehen, von denen in der Fig. 4 die Reibungskörper 94, 95 dargestellt sind. Um eine besonders gute Reibung zwischen den Reibungskörpern 90 bis 95 und der Wand 67 des Verbindungsabschnitts 7 zu erzielen, ist es vorteilhaft, daß die Materialien der Reibungskörper 90 bis 95 auf das Material der Wand 67 des Verbindungsabschnitts 7 abgestimmt sind. Ist beispielsweise die Wand 67 des Verbindungsabschnitts 7 aus einem metallischen Werkstoff gefertigt, dann können die Reibungskörper 90 bis 95 beispielsweise aus einer darauf abgestimmten Gummimischung bestehen. Entsprechende Reibungskörper können auch an den Feststellplatten 65, 66 zur Seite der Wand 67 des Verbindungsabschnitts 7 hin vorgesehen werden. Ein weiterer Vorteil der Reibungskörper 90 bis 95 ist, daß Unebenheiten in der Oberfläche der Feststellplatten 62, 63, 65, 66 und der Innenseite und Außenseite der Wand 67 des Verbindungsabschnitts 7 ausgeglichen werden, da die Anlage nicht ganzflächig erfolgt. In dieser Hinsicht ist es ferner von Vorteil, wenn die Reibungskörper 90 bis 95 aus einem elastischen Material gefertigt sind.

Eine Unebenheit in der Oberfläche der Wand 67 des Verbindungsabschnitts 7 kann insbesondere durch eine Schweißnaht, mit der der Tragarm 5 verschweißt ist, gegeben sein.

Beim Verschieben der Befestigungseinheit 6 in den Richtungen 10, 11 liegen bei horizontaler Einstellung des C-bogenförmigen Tragsarms 5 beide Laufflächen 42, 48 der Laufräder 37, 38 gleichzeitig auf der Lauffläche 36 des Verbindungsabschnitts 7 auf. Dadurch kann insbesondere festgestellt werden, ob sich der Schwerpunkt 16 des beladenen Tragarms 5 bei einer vorgegebenen Neigung des Tragarms 5 zumindest näherungsweise unter dem Aufhängepunkt 26, an dem Träger mittels der Befestigungseinrichtung 6 aufgehängt ist, befindet. Außerhalb der horizontalen Einstellung entsteht, ein Drehmoment um eine Lagerachse des Laufrads 37 bzw. 38 solange, bis einer der Bolzen 60 bzw. 61 in den Aussparungen 8, 9 anliegt.

Die Erfindung ist nicht auf das beschriebene Ausführungsbeispiel beschränkt. Insbesondere kann der Tragarm 5 auch anders, z.B. T-förmig, ausgebildet sein oder aus mehreren Teilen bestehen.

## Patentansprüche

1. Träger (1) zum Tragen von zumindest einem Apparat (2, 3, 4), insbesondere zum Tragen von Bildschirmsicht- und anderen medizinischen Geräten, mit
einem Tragarm (5), an dem der Apparat (2, 3, 4) zumindest mittelbar befestigbar ist, einer Befestigungseinrichtung (6), die zum Aufhängen des Trägers (1) in einen Verbindungsabschnitt (7) des Tragarms (5) eingreift,
**dadurch gekennzeichnet,**
**daß** die Eingriffsstelle (12) der Befestigungseinrichtung (6) so veränderbar ist, dass der Schwerpunkt (16) des beladenen Tragarms (5) bei einer vorgegebenen Neigung des Tragarms (5) zumindest näherungsweise unter den Aufhängepunkt (26) verschiebbar ist, an dem der Träger (1) mittels der Befestigungseinrichtung (6) aufgehängt ist.

2. Träger nach Anspruch 1,
**dadurch gekennzeichnet,**
**daß** der Tragarm (5) bogenförmig, insbesondere C-bogenförmig, ausgebildet ist.

3. Träger nach Anspruch 1 oder 2,
**dadurch gekennzeichnet,**
**daß** die Befestigungseinrichtung (6) ein Gelenk (13), insbesondere ein Kugelgelenk, zur zumindest bezüglich einer Achse (18, 19) drehmomentfreien Aufhängung des Trägers (1) aufweist.

4. Träger nach einem der Ansprüche 1 bis 3,
**dadurch gekennzeichnet,**
**daß** zumindest der Verbindungsabschnitt (7) des Tragarms (5) ein Hohlprofil aufweist, wodurch zumindest im Bereich des Verbindungsabschnittes (7) ein Innenraum (34) ausgebildet ist.

5. Träger nach Anspruch 4,
**dadurch gekennzeichnet,**
**daß** der Verbindungsabschnitt (7) zumindest zwei gegenüberliegende Aussparungen (8, 9) aufweist, durch die die Befestigungseinrichtung (6) in den Innenraum (34) des Verbindungsabschnittes (7) des Tragarms (5) eingreift.

6. Träger nach Anspruch 5,
**dadurch gekennzeichnet,**
**daß** zumindest im wesentlichen in dem Innenraum (34) des Verbindungsabschnittes (7) des Tragarms (5) zum Feststellen der Befestigungseinrichtung (6) bezüglich des Tragarms (5) eine Feststellvorrichtung (35) der Befestigungseinrichtung (6) angeordnet ist.

7. Träger nach Anspruch 6,
**dadurch gekennzeichnet,**
**daß** die Feststellvorrichtung (35) zumindest eine Feststellplatte (62, 63) aufweist, die zum Feststellen von der Seite des Innenraums (34) des Verbindungsabschnittes (7) gegen eine Wand (67) des Verbindungsabschnittes (7) mit einer Feststellkraft beaufschlagbar ist.

8. Träger nach Anspruch 7,
**dadurch gekennzeichnet,**
**daß** der Feststellplatte (62, 63) eine bezüglich der Wand des Verbindungsabschnittes (7) zumindest im wesentlichen gegenüberliegende weitere Feststellplatte (65, 66) zugeordnet ist, wobei zum Feststellen der Befestigungseinrichtung (6) bezüglich des Tragarms (5) die beiden Feststellplatten (62, 65; 63, 66) gegeneinander mit der Feststellkraft beaufschlagbar sind.

9. Träger nach Anspruch 8,
**dadurch gekennzeichnet,**
**daß** die Feststellvorrichtung (35) eine Feststellschraube (81, 83, 83') aufweist, die in ein Gewinde (80, 84) der Feststellplatte (62, 63) eingreift und die sich beim Festziehen zumindest mittelbar an der weiteren Feststellplatte (65, 66) abstützt.

10. Träger nach einem der Ansprüche 1 bis 8,
**dadurch gekennzeichnet,**
**daß** die Eingriffsstelle (12), an der die Befestigungseinrichtung (6) in den Verbindungsabschnitt (7) des Tragarms (5) eingreift, kontinuierlich veränderbar ist.

11. Träger nach Anspruch 9,
**dadurch gekennzeichnet,**
**daß** die Veränderung der Eingriffsstelle (12) mittels einer Spindel (50) erfolgt, die in dem Tragarm (5) gelagert ist und längs der die Befestigungseinrichtung (6) verschiebbar ist.

12. Träger nach einem der Ansprüche 1 bis 10,
**dadurch gekennzeichnet,**
**daß** Zusatzgewichte (24, 25) an dem Tragarm (5) anbringbar sind, um den Schwerpunkt (16) des beladenen Tragarms (5) bei einer vorgegebenen Neigung des Tragarms (5) fein justiert unter den Aufhängepunkt (26) zu verschieben.

## Claims

1. Support (1) to support at least one piece of equipment (2, 3, 4), in particular to support VDUs and other medical equipment, with a support arm (5) on which the equipment (2, 3, 4) can be fixed at least indirectly, and a fixing device (6) which engages with a connecting section (7) of the support arm (5) to suspend the support (1),
**characterized in that**
the engagement point (12) of the fixing device (6) is changeable, to move the centre of gravity (16) of the loaded support arm (5), for a given inclination of the support arm (5), so that it lies at least approximately below the suspension point (26), on which the support (1) is suspended by means of the fixing device (6).

2. Support according to Claim 1,
**characterized in that**
the support arm (5) is bow-shaped, particularly C-bow-shaped.

3. Support according to Claim 1 or 2,
**characterized in that**
the fixing device (6) has a joint. (13), particularly a ball-and-socket joint, for suspension of the support (1) with no torque in relation to at least one axis (18, 19).

4. Support according to one of Claims 1 to 3,
**characterized in that**
at least the connecting section (7) of the support arm (5) has a hollow profile, so that at least in the area of the connecting section (7) an inner space (34) is formed.

5. Support according to Claim 4,
**characterized in that**
the connecting section (7) has at least two recesses (8, 9) which are opposite each other, and through which the fixing device (6) engages with the inner space (34) of the connecting section (7) of the support arm (5).

6. Support according to Claim 5,
**characterized in that**
a locking device (35) for the fixing device (6), to lock the fixing device (6) in relation to the support arm (5), is arranged at least substantially in the inner space (34) of the connecting section (7) of the support arm (5).

7. Support according to Claim 6,
**characterized in that**
the locking device (35) has at least one locking plate (62, 63), to which a locking force can be applied to lock the side of the inner space (34) of the connecting section (7) against one wall (67) of the connecting section (7).

8. Support according to Claim 7,
**characterized in that**
another locking plate (65, 66), which is at least substantially opposite to the locking plate (62, 63) in relation to the wall of the connecting section (7), is assigned to the locking plate (62, 63), it being possible to apply the locking force to both locking plates (62, 65; 63, 66) against each other, to lock the fixing device (6) in relation to the support arm (5).

9. Support according to Claim 8,
**characterized in that**
the locking device (35) has a locking bolt (81, 83, 83'), which engages with a thread (80, 84) of the locking plate (62, 63) and is supported at least indirectly on the further locking plate (65, 66) when it is tightened.

10. Support according to one of Claims 1 to 8,
**characterized in that**
the engagement point (12) at which the fixing device (6) engages with the connecting section (7) of the support arm (5) is continuously changeable.

11. Support according to Claim 9,
**characterized in that**
the engagement point (12) is changed by means of a spindle (50), which is carried in the support arm (5) and along which the fixing device (6) can be moved.

12. Support according to one of Claims 1 to 10,
**characterized in that**
supplementary weights (24, 25) can be attached to the support arm (5), to move the centre of gravity (16) of the loaded support arm (5), for a given inclination of the support arm (5), with fine adjustment below the suspension point (26).

## Revendications

1. Support (1) pour supporter au moins un appareil (2, 3, 4), en particulier pour supporter des appareils d'affichage à écran et autres appareils médicaux, avec
un bras de support (5) auquel l'appareil (2, 3, 4) est fixé au moins indirectement, un dispositif de fixation (6) qui s'engage dans une section de liaison (7) du bras de support (5) pour accrocher le support (1),
**caractérisé en ce que**
le point d'engagement (12) du dispositif de fixation (6) peut être modifié de telle manière que le centre de gravité (16) du bras de support (5) chargé puisse, à une inclinaison prescrite du bras de support (5), être déplacé au moins approximativement jusque sous le point de suspension (26) auquel le support (1) est accroché au moyen du dispositif de fixation (6).

2. Support selon la revendication 1,
**caractérisé en ce que**
le bras de support (5) est réalisé en forme d'arc, en particulier en forme d'arc en C.

3. Support selon la revendication 1 ou 2,
**caractérisé en ce que**
le dispositif de fixation (6) comporte une articulation (13), en particulier une articulation sphérique, pour permettre au moins pour un axe (18, 19) une suspension sans couple du support (1).

4. Support selon une des revendications 1 à 3,
**caractérisé en ce que**
au moins la section de liaison (7) du bras de support (5) comporte un profil creux, grâce à quoi un espace intérieur (34) est réalisé au moins dans la zone de la section de liaison (7).

5. Support selon la revendication 4,
**caractérisé en ce que**
la section de liaison (7) comporte au moins deux évidements opposés (8, 9), à travers lesquels le dispositif de fixation (6) s'engage dans l'espace intérieur (34) de la section de liaison (7) du support (5).

6. Support selon la revendication 5,
**caractérisé en ce que**
au moins pour l'essentiel un dispositif de blocage (35) du dispositif de fixation (6) est disposé dans l'espace intérieur (34) de la section de liaison (7) du bras de support (5) pour fixer le dispositif de fixation (6) par rapport au bras de support (5).

7. Support selon la revendication 6,
**caractérisé en ce que**
le dispositif de blocage (35) comporte au moins une plaque de blocage (62, 63) sur laquelle une force de blocage peut être exercée pour bloquer le côté de l'espace intérieur (34) de la section de liaison (7) contre une paroi (67) de la section de liaison (7).

8. Support selon la revendication 7,
**caractérisé en ce que**
une autre plaque de fixation (65, 66) située au moins pour l'essentiel en face de la paroi de la section de liaison (7) est reliée à la plaque de blocage (62, 63), la force de fixation pouvant être exercée sur les deux plaques de fixation (62, 65 ; 63, 66) pour fixer l'équipement de fixation (6) par rapport au bras de support (5).

9. Support selon la revendication 8,
**caractérisé en ce que**
le dispositif de blocage (35) comporte une vis de blocage (81, 83, 83') qui s'engage dans un filetage (80, 84) de la plaque de fixation (63, 63) et s'appuie lors du serrage au moins indirectement sur l'autre plaque de serrage (65, 66).

10. Support selon une des revendications 1 à 8,
**caractérisé en ce que**
le point d'engagement (12) où le dispositif de fixation (6) s'engage dans la section de liaison (7) du bras de support (5) peut être modifié de manière continue.

11. Support selon la revendication 9,
**caractérisé en ce que**
la modification du point d'engagement (12) est assurée au moyen d'une broche (50) qui est logée dans le bras de support (5) et peut être déplacée le long du dispositif de fixation (6).

12. Support selon une des revendications 1 à 10,
**caractérisé en ce que**
des poids supplémentaires (24, 25) peuvent être placés sur le bras de support (5), afin de déplacer le centre de gravité (16) du bras de support chargé (5) sous le point de suspension (26) grâce à un réglage de précision.
